# EUROPÄISCHE PATENTSCHRIFT

(11) **EP 1 853 335 B1**
(45) Veröffentlichungstag und Bekanntmachung des Hinweises auf die Patenterteilung: **15.10.2008**
(21) Anmeldenummer: 06700026.5
(22) Anmeldetag: 11.01.2006
(51) Int. Cl.: A61M 5/20, A61M 5/24, A61M 5/315, F16F 1/04

(54) **FEDER IN EINEM ODER FÜR EIN INJEKTIONSGERÄT**
SPRING IN OR FOR AN INJECTION APPLIANCE
RESSORT DANS OU POUR UN APPAREIL D'INJECTION

(30) Priorität: 15.04.2005 DE 102005017477; 10.03.2005 DE 202005003847 U; 18.02.2005 DE 202005006333 U
(43) Veröffentlichungstag der Anmeldung: 14.11.2007
(73) Patentinhaber: TecPharma Licensing AG, 3401 Burgdorf (CH)
(72) Erfinder: HOMMANN, Edgar, CH-3257 Grossaffoltern (CH)
(86) Internationale Anmeldenummer: PCT/CH2006/000017
(87) Internationale Veröffentlichungsnummer: WO 2006/086899

(56) Entgegenhaltungen:
- EP-A- 0 577 448
- WO-A-99/30759
- DE-U- 7 340 029
- GB-A- 301 075
- GB-A- 190 916 069
- US-A- 1 817 652
- US-A- 2 866 458
- US-A1- 2001 019 189

## Beschreibung

Die Erfindung betrifft ein Injektionsgerät mit einer Feder, die als Vortriebsfeder einer Fördereinrichtung des Injektionsgeräts dient. Die Erfindung betrifft ferner eine Feder als solche, die als Druckfeder, vorzugsweise Vortriebsfeder, für ein Injektionsgerät verwendet wird. Das Injektionsgerät kann insbesondere ein Injektionspen für die Verabreichung von beispielsweise Insulin, ein Wachstumshormon oder ein Osteoporosepräparat sein.

Mechanische Federn, insbesondere als Druckfedern eingesetzte Schraubenfedern, werden in Injektionsgeräten häufig verwendet, um einen Kolben in einem mit dem zu injizierenden Produkt gefüllten Behältnis in eine Vortriebsrichtung, im Allgemeinen die distale Richtung, vorzutreiben. Die Verwendung als Vortriebsfeder ist insbesondere von Autoinjektionsgeräten bekannt, die für die Erfindung einen bevorzugten Typ von Injektionsgeräten repräsentieren. Vortriebsfedern bekannter Autoinjektoren dienen nicht nur dem Vortrieb des Kolbens, sondern zusätzlich auch dem Vortrieb der Injektionsnadel beim Einstechen. Die Feder entspannt sich bei der Injektion somit nicht nur um die Länge des Kolbenvortriebs, sondern zusätzlich auch um die Länge des Nadelvortriebs. Die Vortriebsfeder eines Autoinjektors ist in solchen Fällen länger als eine Vortriebsfeder, die lediglich dem Kolbenvortrieb dient, wobei der Vergleich natürlich gleiche Längen der jeweils verwendeten Produktbehältnisse unterstellt. Je länger und schlanker die Vortriebsfeder ist, desto kritischer können Biege- oder gar Knickbelastungen sein, zumal im Falle von Autoinjektoren aufgrund des Nadelvortriebs die Feder in Vortriebsrichtung typischerweise eine größere Federkraft aufbringen muss als eine nur dem Vortrieb des Kolbens dienende Feder.

Ein Autoinjektor, wie ihn die Erfindung zwar nicht ausschließlich, aber insbesondere auch betrifft, ist aus der deutschen Patentanmeldung Nr. 103 51 594 bekannt. Bei diesem Autoinjektor ist die Kolbenstange als Hülse mit einem Boden oder einer Schulter an ihrem distalen Ende gebildet, und die Vortriebsfeder ragt in distaler Richtung bis gegen den Boden oder die Schulter in die Hülse. Die Kolbenstangenhülse ist entsprechend der Form des Produktbehältnisses recht schlank, die Feder ist entsprechend noch schlanker. Um ein Knicken oder auch nur Ausbiegen der Feder zu verhindern, bildet die Kolbenstange mit einer Stützstruktur, an der sich die Feder in proximaler Richtung abstützt, eine Teleskopführung für die Feder. Die Kolbenstange und ein Führungsabschnitt der Stützstruktur sind in distaler Richtung gemessen entsprechend lang gestreckt, um eine sichere Führung der Kolbenstange auch dann noch sicherzustellen, wenn die Kolbenstange nach Entleerung des Behältnisses eine distalste Position einnimmt.

Die Patentschrift US-A-2 866 458 offenbart ein Injektionsgerät mit einer Feder, wobei der proximale Teil der Feder einen kleineren Windungsdurchmesser aufweist als der distale Teil.

Es ist eine Aufgabe der Erfindung, die Funktionssicherheit einer Vortriebsfeder eines Injektionsgeräts zu verbessern. Ferner ist es eine Aufgabe der Erfindung, eine für einen Einsatz in einem Injektionsgerät vorgesehene Feder mit verringerter Störanfälligkeit bereitzustellen.

Die Erfindung betrifft ein Gerät für die Injektion eines Produktfluids, das ein Gehäuse, ein von dem Gehäuse gelagertes Produktbehältnis mit einem darin aufgenommenen Kolben, eine Kolbenstange und eine auf die Kolbenstange wirkende Feder aufweist. Die Feder wirkt in distaler Richtung auf die Kolbenstange und diese wiederum auf den Kolben. Die Feder wirkt vorzugsweise als Druckfeder und ist in die distale Richtung lang gestreckt, d. h. ihre in distaler Richtung gemessene axiale Länge ist deutlich größer als ihre rechtwinklig dazu gemessene Breite.

Nach der Erfindung weist die Feder in distaler Richtung nebeneinander wenigstens drei axiale Federabschnitte unterschiedlicher Knickfestigkeit auf, wobei der mittlere Federabschnitt eine größere Knickfestigkeit als die beiden anderen Federabschnitte aufweist. Die Knickfestigkeit des mittleren Federabschnitts ist so groß, dass auf eine Führung der Feder in dem mittleren Federabschnitt verzichtet werden kann und in bevorzugten Ausführungen eine solche auch tatsächlich nicht vorgesehen ist. derartigen Ausführungen führt oder führen die Kolbenstange oder das Behältnis einen distalen Federabschnitt, der insbesondere den ersten Federabschnitt oder vorzugsweise einen von mehreren ersten Federabschnitten bilden kann. Während der mittlere Federabschnitt bei und nach einem Vortrieb der Kolbenstange der Überbrückung eines führungslosen Wegabschnitts der Feder dienen kann, wird der Vortrieb der Kolbenstange zumindest im Wesentlichen von dem ersten Federabschnitt bewirkt, da die Feder in dem ersten Federabschnitt wegen der geringeren Knickfestigkeit weicher ist als in dem mittleren Federabschnitt und dementsprechend der Längenunterschied zwischen dem gespannten und dem entspannten Zustand der Feder im ersten Federabschnitt größer ist als im mittleren.

Vorteilhafte Merkmale der Erfindung werden auch in den Unteransprüchen und deren Kombinationen offenbart.

Ausführungsbeispiele der Erfindung werden nachfolgend anhand von Figuren erläutert. An den Ausführungsbeispielen offenbar werdende Merkmale bilden je einzeln und in jeder Merkmalskombination die Gegenstände der Ansprüche und auch die vorstehend beschriebenen Ausgestaltungen vorteilhaft weiter. Es zeigen:
- Figur 1: ein Injektionsgerät eines ersten Ausführungsbeispiels vor einer Auslösung,
- Figur 2: das Injektionsgerät des ersten Ausführungsbeispiels nach der Auslösung,
- Figur 3: ein Injektionsgerät eines zweiten Ausführungsbeispiels vor einer Auslösung,
- Figur 4: das Injektionsgerät des zweiten Ausführungsbeispiels nach der Auslösung,
- Figur 5: ein Injektionsgerät eines dritten Ausführungsbeispiels vor einer Auslösung und
- Figur 6: das Injektionsgerät des dritten Ausführungsbeispiels nach der Auslösung.

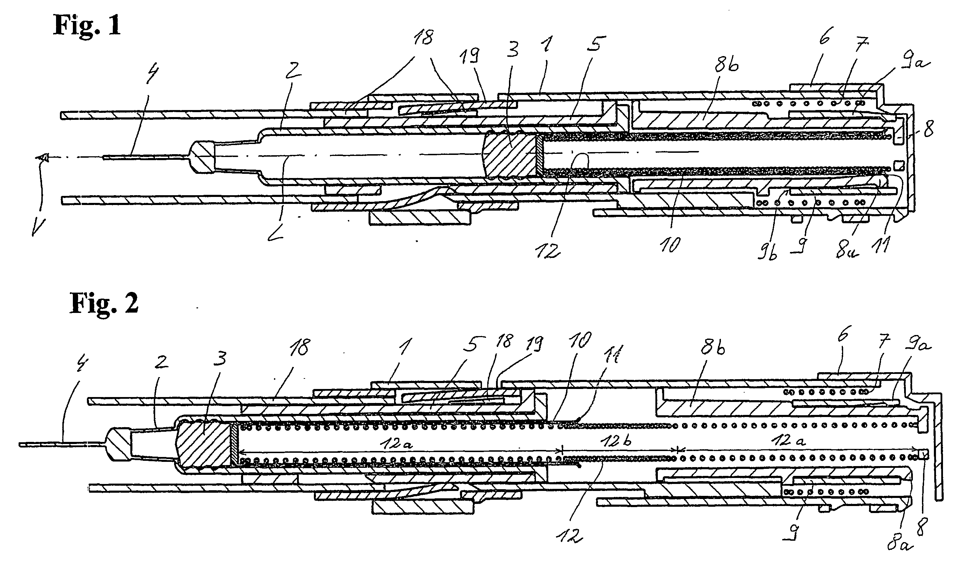

Die Figuren 1 und 2 zeigen ein Injektionsgerät eines ersten Ausführungsbeispiels je im gleichen Längsschnitt, Figur 1 in einem Ausgangszustand vor einer Auslösung und Figur 2 nach der Auslösung. Das Injektionsgerät ist ein Autoinjektor, der nach dem Aufsetzen auf die Injektionsstelle und Auslösung die gesamte Injektion, d. h. das Einstechen einer Injektionsnadel und die Ausschüttung eines zu injizierenden Produkts, automatisch ausführt.

In einem Gehäuse 1 des Injektionsgeräts ist ein mit dem zu injizierenden Produkt gefülltes Behältnis 2 längs einer zentralen Längsachse L des Injektionsgeräts in die distale Richtung V bewegbar aufgenommen. Das Behältnis 2 ist, wie von derartigen Geräten bekannt, als Ampulle gebildet mit einem Auslass an einem distalen Ende und einem Kolben 3, der das Behältnis 2 proximal abdichtet. Der Kolben 3 ist in dem Behältnis 2 längs der Achse L bewegbar aufgenommen, um durch Vortrieb in die distale Richtung V das Produkt durch eine am distalen Ende des Behältnisses 2 angebrachte, in die distale Richtung V vorragende Injektionsnadel 4 auszuschütten. Die Injektionsnadel 4 ist als Kanüle, d. h. mit einem Hohlquerschnitt, gebildet, könnte grundsätzlich aber auch einen Vollquerschnitt mit wenigstens einem Strömungskanal an der Umfangsfläche haben. Die Injektionsnadel 4 wird bis über ihre distale Spitze von einem Nadelschutz 18 umgeben. Der Nadelschutz 18 bildet sowohl einen Sichtschutz, d.h. er verdeckt die Sicht auf die Injektionsnadel 4, als auch einen Schutz gegen Durchgriff auf die Injektionsnadel 4.

Das Behältnis 2 ist in einem Behältnishalter 5 aufgenommen. Der Behältnishalter 5 umgibt das Behältnis 2 eng anliegend. Das Gehäuse 1 führt den Behältnishalter 5 axial bewegbar. Das Behältnis 2 hintergreift mit einem am proximalen Ende gebildeten Behältnisflansch den Behältnishalter 5, so dass eine in die distale Richtung V auf das Behältnis 2 wirkende Kraft auf den Behältnishalter 5 übertragen wird.

Eine Fördereinrichtung des Injektionsgeräts umfasst über den Kolben 3 hinaus eine Kolbenstange 10 und eine Vortriebsfeder 12. Die Vortriebsfeder 12 ist eine Schraubenfeder und wirkt nach Auslösung des Injektionsgeräts als Druckfeder in die distale Richtung V auf die Kolbenstange 10, welche die Federkraft auf den Kolben 3 überträgt. Die Vortriebsfeder 12 ragt in die distale Richtung V in die hohlzylindrische Kolbenstange 10 bis gegen einen Boden der Kolbenstange 10. Die Kolbenstange 10 ist als schlanke, dünnwandige Hülse mit einem ebenfalls dünnen Boden gebildet. Der Boden könnte auch durch eine schmale, nach einwärts ragende Schulter ersetzt werden; es muss nur eine sichere Abstützung der Vortriebsfeder 12 gewährleistet sein. Der Boden ist mit der Rückseite des Kolbens 3 in einem losen Anschlagkontakt. In die proximale Richtung stützt sich die Vortriebsfeder 12 an einer Stütz- und Führungsstruktur 8 ab. Die Vortriebsfeder 12 ist im gespannten Zustand über nahezu ihre gesamte Länge in der Kolbenstange 10 aufgenommen und überragt die Kolbenstange 10 in proximaler Richtung nur so weit es für ihre Abstützung erforderlich ist.

Die Vortriebsfeder 12 wird mittels einer Rückhalteeinrichtung im gespannten Zustand gehalten. Die Rückhalteeinrichtung ist auslösbar. Sie umfasst ein Blockierglied 6, eine Feder 7, die Stütz- und Führungsstruktur 8, einen Schnapper 9a und ein fest mit der Kolbenstange 10 verbundenes Rückhalteelement 11. Das Rückhalteelement 11 ist als schräg nach außen weisender Flansch am Ende der Hülsenrand der Kolbenstange 10 geformt. Das Rückhalteelement 11 hintergreift im Ausgangszustand des Geräts eine elastische Biegezunge 8a der Stütz- und Führungsstruktur 8. Die Biegezunge 8a ist im Ausgangszustand elastisch nach radial einwärts in den Eingriff mit dem Rückhalteelement 11 gebogen. Ein Zurückschnappen wird durch eine Gegenfläche einer Schnapperhülse 9 verhindert, die den Schnapper 9 ebenfalls als elastische Biegezunge bildet. Die Schnapperhülse 9 ist axial unbeweglich mit dem Gehäuse 1 verbunden und umgibt die Stütz- und Führungsstruktur 8. Ihr Schnapper 9a weist an seinem proximalen Ende nach radial einwärts eine Verdickung auf, mit der er in eine Ausnehmung an einer Mantelaußenfläche der Stütz- und Führungsstruktur 8 greift. Das hülsenförmige Blockierglied 6 blockiert den Schnapper 9a in diesem Eingriff. Die Feder 7 wirkt in proximaler Richtung auf das Blockierglied 6. Das Blockierglied 6 ist im Ausgangszustand in der den Schnapper 9a blockierenden Position arretiert. Die Arretierung ist durch Betätigung eines Auslöseelements lösbar.

Um das Injektionsgerät auszulösen, wird das Auslöseelement betätigt und dadurch die Arretierung des Blockierglieds 6 gelöst. Das Blockierglied 6 bewegt sich aufgrund der Kraft der Feder 7 relativ zu dem Gehäuse 1, der Stütz- und Führungsstruktur 8 und dem Schnapper 9a in die proximale Richtung und gibt den Schnapper 9a frei. Da auf die Stütz- und Führungsstruktur 8 die Kraft der gespannten Vortriebsfeder 12 wirkt, drückt die Stütz- und Führungsstruktur 8 in die proximale Richtung gegen den zwar noch im Eingriff befindlichen, aber bereits vom Blockierglied 6 freien Schnapper 9a, so dass dieser elastisch nach außen gebogen wird und der Eingriff zwischen dem Schnapper 9a und der Stütz- und Führungsstruktur 8 sich löst. Die Ausnehmung der Stütz- und Führungsstruktur 8 und der in die Ausnehmung eingreifende Abschnitt des Schnappers 9a sind so geformt, dass der eingreifende Abschnitt durch die in die proximale Richtung wirkende Druckkraft der Stütz- und Führungsstruktur 8 aus dem Eingriff gleiten kann, indem der Schnapper 9a nach außen gebogen wird. Nach Lösung des Eingriffs drückt die Vortriebsfeder 12 in der Anfangsphase nach dem Auslösen die Stütz- und Führungsstruktur 8 in die proximale Richtung bis gegen einen von der Schnapperhülse 9 gebildeten Anschlag 9b. Wenn die Stütz- und Führungsstruktur 8 auf Anschlag gelangt ist, gegebenenfalls auch bereits kurz vor der Bewegung in die Anschlagposition, schnappt die bis dahin in dem Eingriff mit dem Rückhalteelement 11 befindliche Biegezunge 8a nach radial außen aus dem Eingriff, so dass die Kolbenstange 10 freikommt. Nun kann die Vortriebsfeder 12 die Kolbenstange 10 relativ zu dem Gehäuse 1 in die distale Richtung V drücken. Wegen der zwischen dem Kolben 3 und der Seitenwand des Behältnisses 2 wirkenden Haftreibung wird in der sich nun anschließenden zweiten Bewegungsphase nach Auslösung das Behältnis 2 und damit gemeinsam der Behältnishalter 5 in die distale Richtung V bewegt. Bei dieser Bewegung sticht die Injektionsnadel 4 aus dem Gehäuse 1 bis in eine distalste Position hervor. Die Vorstechbewegung wird durch Anschlag des Behältnishalters 5 an einen Gegenanschlag begrenzt. Spätestens nach Anschlag des Behältnishalters 5 an den Gegenanschlag wird die Haftreibung zwischen dem Kolben 3 und dem Behältnis 2 überwunden, und die Vortriebsfeder 12 drückt in der letzten, dritten Bewegungsphase nach Auslösung den Kolben 3 im Behältnis 2 in die distale Richtung V vor, so dass das Produkt ausgeschüttet wird. Die vorstehend geschilderten Bewegungsphasen können ohne Überlappung nacheinander oder auch einander zum Teil zeitlich überlappend stattfinden. Die Lagerung der bewegten Teile ist idealerweise so gestaltet, dass der Förderhub des Kolbens 3 erst beginnt, wenn die Einstechbewegung der Injektionsnadel 4 beendet ist.

Figur 2 zeigt das Injektionsgerät nach der Injektion. Das Behältnis 2 ist entleert. Der Kolben 3 und die Injektionsnadel 4 nehmen je ihre distalste Position ein. Die Vortriebsfeder 12 ist von einer für den Vortrieb des Kolbens 3 erforderlichen Mindestspannung abgesehen entspannt. Sie erstreckt sich in diesem Zustand der Restspannung über den größten Teil der vom distalen Ende des Gehäuses 1 bis zum proximalen Ende des Injektionsgeräts gemessenen Gesamtlänge des Injektionsgeräts.

Nach der Injektion und nachdem die Injektionsnadel 4 aus dem Körpergewebe gezogen und das Injektionsgerät von der Einstechstelle abgehoben wurden, schnappt der Nadelschutz 18 relativ zu dem Behältnishalter 5 und somit auch relativ zu der Injektionsnadel 4 in die distale Richtung, bis in eine Schutzposition, in welcher der Nadelschutz 18 die Injektionsnadel 4 wieder bis über deren Spitze hinaus umgibt. Der Nadelschutz 18 ist in der Schutzstellung, wie im übrigen auch in der Stellung vor Auslösung des Injektionsgeräts, verriegelt, so dass er sich nicht gegenüber der Injektionsnadel 4 in die proximale Richtung bewegen kann. In der Schutzstellung nach Injektion wird die Verriegelung durch Anschlagkontakt eines proximalen Endes des Nadelschutzes 18 und eines distalen Endes eines Verriegelungselements 19 bewirkt, indem sich der Nadelschutz 18 nach Herausziehen der Injektionsnadel 4 und der damit einhergehenden Druckentlastung unter Einwirkung einer nicht dargestellten Feder mit seinem proximalen Ende vor das Verriegelungselement 19 bewegt. Vor der Injektion wird diese Bewegung des Nadelschutzes 18 durch einen sich während der Bewegung des Behältnisses 2 automatisch lösenden Blockiermechanismus verhindert.

Wie in Figur 2 zu erkennen ist, weist die Vortriebsfeder 12 axiale Federabschnitte 12a und 12b auf, in denen die Federwindungen unterschiedlich dicht beieinander liegen. In dem mittleren Federabschnitt 12b liegen im Zustand der Restspannung sämtliche Federwindungen in axialer Richtung gegeneinander auf Block, während die Federwindungen in den beiden äußeren Federabschnitten 12a voneinander beabstandet sind. Die beiden äußeren Federabschnitte 12a unterscheiden sich voneinander lediglich hinsichtlich ihrer Länge. Durch die im mittleren Federabschnitt 12b dichter nebeneinander liegenden Federwindungen wird über die Länge des Federabschnitts 12b eine größere Knickfestigkeit als in den beiden äußeren Federabschnitten 12a erzielt, wobei deren Knickfestigkeit für den Vergleich je für Teilabschnitte mit der Länge des mittleren Federabschnitts 12b zu bestimmen ist. Die erhöhte Knickfestigkeit geht mit einer höheren Federsteifigkeit einher, so dass die für den Vortrieb des Kolbens 3 und der Injektionsnadel 4 erforderliche Federarbeit in den beiden äußeren axialen Federabschnitten 12a geleistet wird. Dort dehnt sich die Vortriebsfeder 12 bei ihrer Entspannung axial aus. Im Ausführungsbeispiel, in dem die Federwindungen in dem mittleren Federabschnitt 12b bei in distalster Position befindlichem Kolben 3 noch auf Block liegen, wird die gesamte Federarbeit in den beiden äußeren Federabschnitten 12a geleistet. Grundsätzlich wäre für andere Ausführungen jedoch auch denkbar, dass sich die Vortriebsfeder 12 in dem mittleren Federabschnitt 12b entspannt, allerdings weniger als in den äußeren Federabschnitten 12a und auf Kosten der Knickfestigkeit.

In den Federabschnitten 12a wird die Vortriebsfeder 12 während der gesamten Vortriebsbewegung geführt oder zumindest doch gesichert. Eine Führung im engeren Sinne bildet die Kolbenstange 10, da die Federwindungen an deren Mantelinnenfläche anliegen. Die Stütz- und Führungsstruktur 8 bildet nur eine Führung im weiteren Sinne, d. h. sie bildet nur eine Sicherung gegen ein übermäßiges Biegen oder gar Knicken des proximalen Federabschnitts 12a. Hierfür bildet die Stütz- und Führungsstruktur 8 einen Führungsabschnitt 8b, an dessen Mantelinnenfläche, die Kolbenstange 10 über ihr Rückhalteelement 11 geführt wird. Entsprechend besteht zwischen der Vortriebsfeder 12 und dem Führungsabschnitt 8b nach dem Ausfahren der Kolbenstange 10 ein kleiner radialer Abstand, der jedoch für eine Sicherung der Vortriebsfeder 12 gegen Biegung ausreicht.

Wenn die Kolbenstange 10 ihre in Figur 2 gezeigte distalste Position einnimmt, ist sie vollständig aus dem Führungsabschnitt 8b ausgefahren, und es besteht ein axial lichter Abstand zwischen der Kolbenstange 10 und der Stütz- und Führungsstruktur 8. Die Vortriebsfeder 12 wird über die gesamte Länge dieses Abstands weder geführt noch sonst wie gegen Biegen oder gar Knicken gesichert. Der Federabschnitt 12b überbrückt diesen Abstand, so dass auch über dessen Länge ein übermäßiges Biegen oder gar Knicken der Vortriebsfeder 12 verhindert wird. Zur Erhöhung der Sicherheit ragt der Federabschnitt 12b in die distale Richtung V ein kleines Stück weit in die Kolbenstange 10 und in die proximale Richtung ein ebenfalls kleines Stück weit in die Führung 8b. Die Sicherheit gegen Biegen und Knicken wird dadurch weiter erhöht.

Zur Vortriebsfeder 12 des Ausführungsbeispiels sei noch angemerkt, dass sie über ihre gesamte Länge aus dem gleichen Federdraht gewickelt ist, der überall den gleichen Querschnitt und die gleichen Materialeigenschaften aufweist. Die erhöhte Knickfestigkeit geht somit ausschließlich auf die geringere Steigung der Windungen im Federabschnitt 12b zurück. Die Steigung in den Federabschnitten 12a ist überall die gleiche. Auch im Federabschnitt 12b ist die Steigung konstant. In dem mittleren Federabschnitt 12b ist somit auch überall die Biegesteifigkeit größer als in den äußeren Federabschnitten 12a.

Der axiale Abstand zwischen der Kolbenstange 10 und der Stütz- und Führungsstruktur 8 entspricht derjenigen Länge, um die das Injektionsgerät gegenüber einem ansonsten gleichen Injektionsgerät verkürzt ist, bei dem die Vortriebsfeder im entspannten Zustand in herkömmlicher Weise über ihre gesamte Länge geführt oder gesichert wird.

Falls der Behältnishalter 5 oder gegebenenfalls unmittelbar das Behältnis 2 in deren distalsten Position nicht gegen eine Bewegung in die proximale Richtung relativ zu dem Gehäuse 1 verriegelt sein sollten, wird durch die besondere Knickfestigkeit der Feder 13 verhindert, dass die Feder 13 knickt, sollte auf den Nadelschutz 18 eine in die proximale Richtung gerichtete Kraft ausgeübt werden, die über den Anschlagkontakt von Nadelschutz 18 und Verriegelungselement 19 und dessen Anschlagkontakt mit dem Behältnishalter 5 auf das Behältnis 2 und schließlich auf die Feder 13 wirkt. Die Knickfestigkeit der Feder 13 ist aber auch vorteilhaft allein schon während der Einstechbewegung der Injektionsnadel 4 und der Förderbewegung des Kolbens 3.

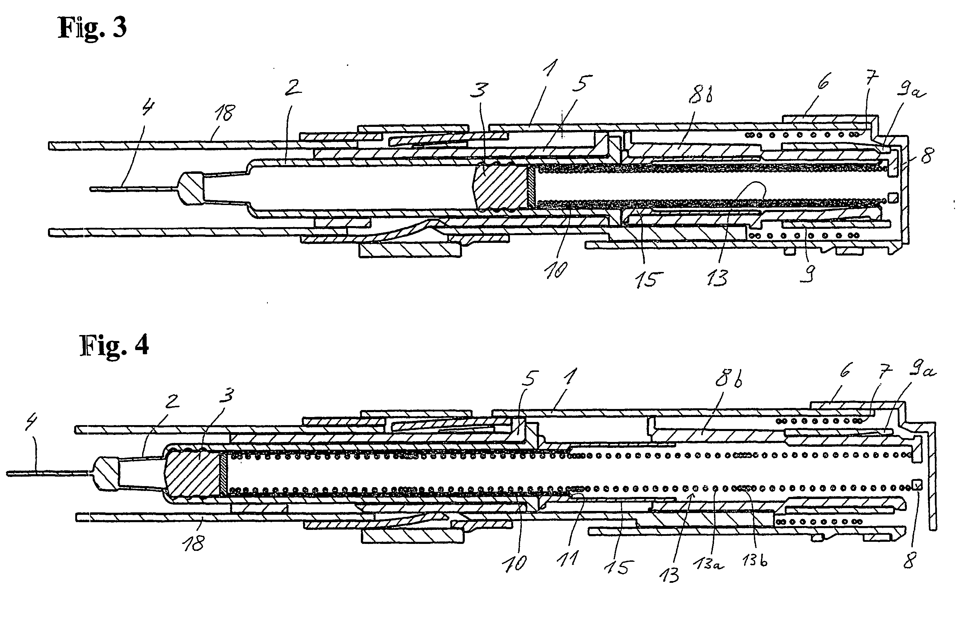

Die Figuren 3 und 4 zeigen ein Injektionsgerät eines zweiten Ausführungsbeispiels, in dem der axiale Abstand nach dem Ausfahren der Kolbenstange 10 von einer Führungsstruktur 15 überbrückt wird, die dort ein übermäßiges Biegen oder gar Knicken der Vortriebsfeder 13 des zweiten Ausführungsbeispiels verhindert. Figur 3 zeigt das Injektionsgerät wieder im Ausgangszustand vor der Auslösung, und Figur 4 zeigt das Gerät nach der Auslösung und Entleerung des Behältnisses 2.

Die Vortriebsfeder 13 weist axial alternierend nebeneinander Federabschnitte 13a und 13b auf, wobei die Knickfestigkeit der Federabschnitte 13b größer ist als die der Federabschnitte 13a, bezogen je auf gleiche Längen. Die Federabschnitte 13a und 13b sind jeweils kürzer als die entsprechenden Federabschnitte 12a und 12b des ersten Ausführungsbeispiels. Insbesondere sind die knickfesteren Federabschnitte 13b deutlich kürzer als der axiale Abstand zwischen der Stützstruktur 8 und der in distalster Position befindlichen Kolbenstange 10, so dass zur sicheren Verhinderung eines übermäßigen Biegens oder gar Knickens der Vortriebsfeder 13 in diesem bereich die Führungsstruktur 15 vorgesehen ist.

Von der Vortriebsfeder 13 und der Führungsstruktur 15 sowie einer damit in Verbindung stehenden Modifikation des Führungsabschnitts 8b abgesehen entspricht das Injektionsgerät des zweiten Ausführungsbeispiels dem des ersten Ausführungsbeispiels, und es werden dementsprechend für die Komponenten des Injektionsgeräts die Bezugszeichen des ersten Ausführungsbeispiels verwendet. Auf die dortigen Ausführungen sei daher verwiesen.

Die Führungsstruktur 15 ist eine einteilige, in sich steife Hülse, die auf der Mantelaußenfläche der Kolbenstange 10 in einem Gleitsitz gelagert ist. Sie weist zwei ineinander übergehende Hülsenabschnitte auf, nämlich einen distalen Abschnitt mit einem ersten Innendurchmesser und einen proximalen Hülsenabschnitt mit einem demgegenüber größeren, zweiten Innendurchmesser. Der proximale Abschnitt ist länger als der distale Abschnitt. Mit ihrem distalen Hülsenabschnitt sitzt die Führungsstruktur im Gleitkontakt auf der Kolbenstange 10. Ferner kontaktiert sie mit ihrem distalen Ende im Ausgangszustand und zumindest auch bei in distalster Position befindlicher Kolbenstange 10 in distaler Richtung V die Rückseite des Behältnisses 2. Die Mantelaußenfläche der Führungsstruktur 15 ist glatt und steht mit einer Mantelinnenfläche des Führungsabschnitts 8b in Gleitkontakt. Grundsätzlich könnte zwischen der Führungsstruktur 15 und dem Führungsabschnitt 8b jedoch auch ein Ringspalt gebildet sein.

Im Ausgangszustand überlappt die Kolbenstange 10 vollständig die Führungsstruktur 15. Ferner ist die Führungsstruktur 15 zumindest im Wesentlichen in der Stütz- und Führungsstruktur 8 aufgenommen. Die Führungsstruktur 15 erfordert somit keine Verlängerung des Injektionsgeräts.

Durch Auslösung des Injektionsgeräts wird der anhand des ersten Ausführungsbeispiels beschriebene Bewegungsablauf in Gang gesetzt. Der Gleitsitz der Führungsstruktur 15 auf der Kolbenstange 10 ist vorzugsweise so gestaltet, dass die Führungsstruktur 15 während der Vortriebsbewegung des Behältnisses 2 von der Kolbenstange 10 mitgenommen wird und stets im Kontakt mit dem Behältnis 2 bleibt. Nachdem der Behältnishalter 5 seine Anschlagposition erreicht hat, drückt die Kolbenstange 10 in der letzten Bewegungsphase den Kolben 3 in die distale Richtung V, bis das Behältnis 2 entleert ist. Während der letzten Bewegungsphase bewegt sich die Kolbenstange 10 im Gleitkontakt relativ zu der Führungsstruktur 15, bis sie ihre distalste Position erreicht hat. Die Führungsstruktur 15 bildet durch den Übergang zwischen dem distalen Abschnitt und dem proximalen Abschnitt eine Mitnahmeschulter, gegen die das Rückhalteelement 11 der Kolbenstange 10 zumindest in der distalsten Position der Kolbenstange 10 auf Anschlag gelangt. Durch diesen Mitnahmeeingriff wird sichergestellt, dass die Führungsstruktur 15 auch im Falle eines nicht optimal gestalteten Gleitsitzes bei der Injektion soweit in die distale Richtung V bewegt wird, dass sie den sich ergebenden Abstand zwischen der Kolbenstange 10 und der Stütz- und Führungsstruktur 8 überbrückt.

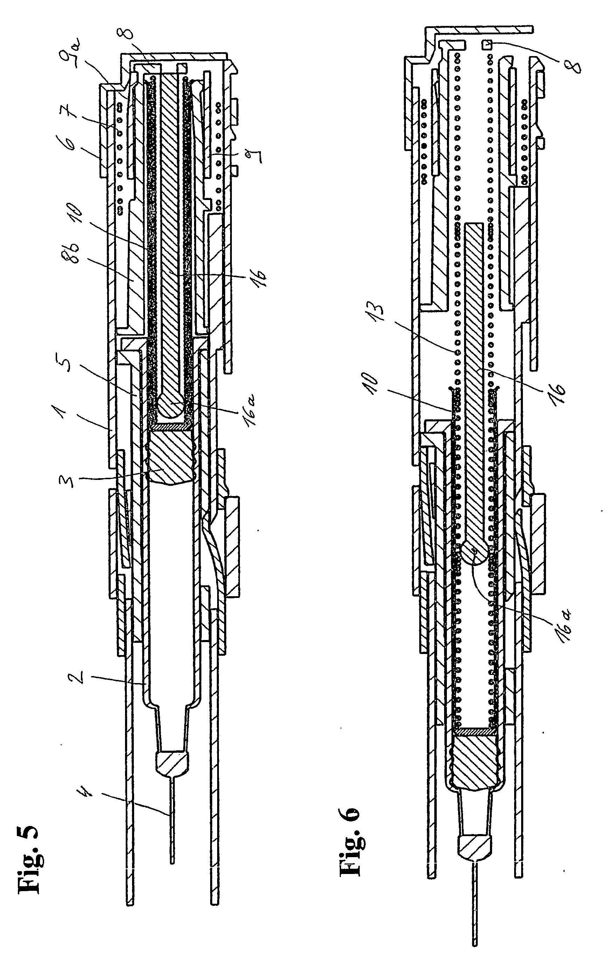

Ein drittes Ausführungsbeispiel eines Injektionsgeräts ist in den Figuren 5 und 6 dargestellt. Das Injektionsgerät des dritten Ausführungsbeispiels weist eine Führungsstruktur 16 auf, die im Ausgangszustand des Geräts innerhalb der Kolbenstange 10 aufgenommen ist. Von der Führungsstruktur 16 abgesehen entspricht das Injektionsgerät des dritten Ausführungsbeispiels dem Injektionsgerät des zweiten Ausführungsbeispiels, so dass auf die Ausführungen hierzu verwiesen sei.

Die Führungsstruktur 16 ist als Führungsstab gebildet. Sie weist etwa die Länge der gespannten Vortriebsfeder 13 auf. An dem distalen Ende der Führungsstruktur 16 ist ein Eingriffselement. 16a geformt. Das Eingriffselement 16a ist eine Verdickung, im Ausführungsbeispiel eine kugelförmige Verdickung, des Führungsstabs. Das Eingriffselement 16a ist in einem Presskontakt mit der Vortriebsfeder 13. Die rechtwinklig zu der Längsachse L gemessene Dicke des Eingriffselements 16a ist so bemessen, dass die Windungen der Vortriebsfeder 13 bei einer Vortriebsbewegung der Kolbenstange 10 zwar in die distale Richtung V über das Eingriffselement 16a gleiten können, die Gleitbewegung aber doch in solch einem Ausmaß erschwert ist, dass die Führungsstruktur 16 über einen Teil der von der Kolbenstange 10 bis in die distalste Position zurückgelegten Wegstrecke mitgenommen wird. Das Eingriffselement 16a greift geringfügig in den Zwischenraum axial benachbarter Federwindungen ein. Bei in distalster Position befindlicher Kolbenstange 10 überbrückt die Führungsstruktur 16 den axial lichten Abstand zwischen der Kolbenstange 10 und der Stütz- und Führungsstruktur 8, so dass auch im dritten Ausführungsbeispiel eine ausreichende Sicherheit gegen Biegen oder gar Knicken gewährleistet ist, nämlich durch eine Führung, d. h. im engeren Sinne nur eine Sicherung, innerhalb der Vortriebsfeder 13.

### Bezugszeichen:

- 1: Gehäuse
- 2: Behältnis
- 3: Kolben
- 4: Injektionsnadel
- 5: Behältnishalter
- 6: Blockierglied
- 7: Feder
- 8: Stütz- und Führungsstruktur
- 8a: Biegezunge
- 8b: Führungsabschnitt
- 9: Schnapperhülse
- 9a: Schnapper
- 9b: Anschlag
- 10: Kolbenstange
- 11: Rückhalteelement
- 12: Feder
- 12a: erster Federabschnitt
- 12b: zweiter Federabschnitt
- 13: Feder
- 13a: erster Federabschnitt
- 13b: zweiter Federabschnitt
- 14: -
- 15: Führungsstruktur
- 16: Führungsstruktur
- 16a: Eingriffselement
- 17: -
- 18: Nadelschutz
- 19: Verriegelungselement

- L: Längsachse
- V: distale Richtung, Vortriebsrichtung

## Patentansprüche

1. Injektionsgerät umfassend:
a) ein Gehäuse (1)
b) ein von dem Gehäuse (1) aufgenommenes Produktbehältnis (2), in dem ein Kolben (3) in eine distale Richtung (V) bewegbar aufgenommen ist, um ein Produkt auszuschütten.
c) eine in die distale Richtung (V) auf den Kolben (3) wirkende Kolbenstange (10)
d) eine in die distale Richtung (V) auf die Kolbenstange (10) wirkende Feder (12;13), wobei die Feder (12; 13) in distaler Richtung (V) nebeneinander wenigstens zwei Federabschnitte (12a,12b;13a,13b) umfasst, die bei in distalster Position befindlicher Kolbenstange (10) bezogen auf je die gleiche Länge eine unterschiedliche Knickfestigkeit aufweisen,
**dadurch gekennzeichnet, dass**
e) die Feder (12;13) wenigstens drei axiale Federabschnitte (12a,12b;13a,13b) nebeneinander aufweist, und der mittlere Federabschnitt (12b;13b) knickfester als die beiden anderen Federabschnitte (12a;13a) ist

2. Injektionsgerät nach Anspruch 1, **dadurch gekennzeichnet, dass** von den wenigstens drei Federabschnitten (12a,12b;13a,13b) der die grössere Knickfestigkeit aufweisende Federabschnitt (12b; 13b) einen proximalen Endabschnitt der Kolbenstange (10) überlappt oder in die distale Richtung (V) bis an das proximale Ende der Kolbenstange (10) reicht, wenn die Kolbenstange (10) eine distalste Position einnimmt.

3. Injektionsgerät nach Anspruch 1 oder 2, **dadurch gekennzeichnet, dass** die Feder eine Schraubenfeder mit um eine Federachse spiralig nebeneinander umlaufenden Windungen ist.

4. Injektionsgerät nach Anspruch 3, **dadurch gekennzeichnet, dass** die Windungen Im entspannten Zustand der Feder (12,13) in dem die grössere Knickfestigkeit aufweisenden Federabschnitt (12b; 13b) dichter nebeneinander angeordnete sind als in dem die kleinere Knickfestigkeit aufweisenden Federabschnitt (12a; 13b).

5. Injektionsgerät nach Anspruch 3 oder 4, **dadurch gekennzeichnet, dass** die Windungen in dem die grössere Knickfestigkeit aufweisenden Federabschnitt (12b;13b) bei in distalster Position befindlicher Kolbenstange (10) aneinander auf Block liegen.

6. Injektionsgerät nach einem der Ansprüche 3 bis 4, **dadurch gekennzeichnet, dass** die Steigung der Windungen innerhalb der Federabschnitte (12a,12b;13a,13b) jeweils konstant ist.

7. Injektionsgerät nach einem der vorhergehenden Ansprüche, **dadurch gekennzeichnet, dass** der die grössere Knickfestigkeit aufweisende Federabschnitt (12b;13b) über seine axiale Länge überall eine grössere Biegefestigkeit als der die kleiner Knickfestigkeit aufweisende Federabschnitt (12a; 13a) aufweist.

8. Injektionsgerät nach einem der vorhergehenden Ansprüche, **dadurch gekennzeichnet, dass** ein Führungsabschnitt (8b) vorgesehen ist, der in einer proximalsten Position der Kolbenstange (10) die Kolbenstange (10) überlappt, und dass die Kolbenstange (10) bei dem Vortrieb in die distale Richtung (V) aus der Überlappung mit dem Führungsabschnitt (8) gelangt und ein proximales Ende der Kolbenstange (10) in einer distalsten Position der Kolbenstange (10) axial von dem Führungsabschnitt (8b) beabstandet ist.

9. Injektionsgerät nach einem der vorhergehenden Ansprüche, **dadurch gekennzeichnet, dass** das Gehäuse (1) das Produktbehältnis (2) in die distale Richtung (V) bewegbar lagert und dass die Feder (12; 13) in die distale Richtung (V) auf das Produktbehältnis (2) wirkt, vorzugsweise über den Kolben (3).

## Claims

1. An injection device comprising:
a) a housing (1),
b) a product container (2) which is accommodated by the housing (1) and in which a piston (3) is accommodated movably in a distal direction (V) to expel a product,
c) a piston rod (10) acting on the piston (3) in the distal direction (V), and
d) a spring (12; 13) acting on the piston rod (10) in the distal direction (V), wherein the spring (12; 13) includes in mutually juxtaposed relationship in the distal direction (V) at least two spring portions (12a, 12b; 13a, 13b) which have a different buckling strength when the piston rod (10) is in the distal position in relation to the same respective length,
**characterised in that**
e) the spring (12; 13) has at least three axial spring portions (12a, 12b; 13a, 13b) in mutually juxtaposed relationship and the central spring portion (12b; 13b) is stronger in respect of buckling than the other two spring portions (12a; 13a).

2. An injection device according to claim 1 **characterised in that** of the at least three spring portions (12a, 12b; 13a, 13b) the spring portion (12b; 13b) having the greater buckling strength overlaps a proximal end portion of the piston rod (10) or extends in the distal direction (V) to the proximal end of the piston rod (10) when the piston rod (10) assumes a most distal position.

3. An injection device according to claim 1 or claim 2 **characterised in that** the spring is a coil spring with turns peripherally extending in a spiral in mutually juxtaposed relationship about a spring axis.

4. An injection device according to claim 3 **characterised in that** in the relaxed condition of the spring (12, 13) the turns in the spring portion (12b; 13b) having the greater buckling strength are arranged in more closely mutually adjacent relationship than in the spring portion (12a; 13a) having the lower buckling strength.

5. An injection device according to claim 3 or claim 4 **characterised in that** the turns in the spring portion (12b; 13b) having the greater buckling strength are in a blocked condition against each other when the piston rod (10) is in the most distal position.

6. An injection device according to one of claims 3 and 4 **characterised in that** the pitch of the turns within the spring portions (12a, 12b; 13a, 13b) is respectively constant.

7. An injection device according to one of the preceding claims **characterised in that** the spring portion (12b; 13b) having the greater buckling strength is everywhere of a greater bending strength over its axial length than the spring portion (12a; 13a) having the lower buckling strength.

8. An injection device according to one of the preceding claims **characterised in that** there is provided a guide portion (8b) which in a most proximal position of the piston rod (10) overlaps the piston rod (10) and that the piston rod (10) passes out of the overlap with the guide portion (8) in the forward drive movement in the distal direction (V) and a proximal end of the piston rod (10) is axially spaced from the guide portion (8b) in a most distal position of the piston rod (10).

9. An injection device according to one of the preceding claims **characterised in that** the housing (1) supports the product container (2) movably in the distal direction (V) and the spring (12; 13) acts on the product container (2) in the distal direction (V), preferably by way of the piston (3).

## Revendications

1. Appareil d'injection comprenant :
a) un boîtier (1)
b) un récipient pour produit (2) logé dans le boîtier (1), récipient dans lequel est logé un piston (3) de manière à se déplacer dans une direction distale (V), afin de déverser un produit,
c) une tige de piston (10) agissant sur le piston (3) dans la direction distale (V),
d) un ressort (12 ; 13) agissant sur la tige de piston (10) dans la direction distale (V), le ressort (12 ; 13) comprenant dans la direction distale (V) au moins deux sections de ressort (12a, 12b ; 13a, 13b) côte à côte, lesquelles présentent différentes résistances au flambage, par rapport à la même longueur, lorsque la tige de piston (10) se trouve dans la position la plus distale,
**caractérisé en ce que**
e) le ressort (12 ; 13) présente au moins trois sections axiales de ressort (12a, 12b; 13a, 13b) côte à côte, et la section médiane de ressort (12b ; 13b) est plus résistante au flambage que les deux autres sections de ressort (12a ; 13a).

2. Appareil d'injection selon la revendication 1, **caractérisé en ce que** parmi les au moins trois sections de ressort (12a, 12b ; 13a, 13b) la section de ressort (12b ; 13b) présentant la plus grande résistance au flambage recouvre une section d'extrémité proximale de la tige de piston (10), ou s'étend dans la direction distale (V) jusqu'à l'extrémité proximale de la tige de piston (10), lorsque la tige de piston (10) prend une position distale maximale.

3. Appareil d'injection selon la revendication 1 ou 2, **caractérisé en ce que** le ressort est un ressort à boudin muni de spires s'étendant côte à côte en spirale autour d'un axe de ressort.

4. Appareil d'injection selon la revendication 3, **caractérisé en ce que** les spires, à l'état relâché du ressort (12, 13) sont disposées plus près les unes des autres dans la section de ressort (12b; 13b) présentant la plus grande résistance au flambage que dans la section de ressort (12a ; 13a) présentant la plus faible résistance au flambage.

5. Appareil d'injection selon la revendication 3 ou 4, **caractérisé en ce que** les spires sont juxtaposées de manière à former un bloc dans la section de ressort (12b ; 13b) présentant la plus grande résistance au flambage lorsque la tige de piston (10) se trouve dans la position la plus distale.

6. Appareil d'injection selon l'une quelconque des revendications 3 à 4, **caractérisé en ce que** le pas des spires à l'intérieur des sections de ressort (12a, 12b ; 13a, 13b) est respectivement constant.

7. Appareil d'injection selon l'une quelconque des revendications précédentes, **caractérisé en ce que** la section de ressort (12b ; 13b) présentant la plus grande résistance au flambage présente en tout point sur sa longueur axiale une plus grande résistance à la flexion que la section de ressort (12a ; 13a) présentant la plus faible résistance au flambage.

8. Appareil d'injection selon l'une quelconque des revendications précédentes, **caractérisé en ce qu'**il est prévu une section de guidage (8b) qui recouvre la tige de piston (10) dans une position proximale maximale de la tige de piston (10), et **en ce que** la tige de piston (10) sort du recouvrement avec la section de guidage (8) lors de la poussée en avant dans la direction distale (V), et une extrémité proximale de la tige de piston (10) est distante axialement de la section de guidage (8b) dans une position distale maximale de la tige de piston (10).

9. Appareil d'injection selon l'une quelconque des revendications précédentes, **caractérisé en ce que** le boîtier (1) loge le récipient pour produit (2) de manière mobile dans la direction distale (V) et **en ce que** le ressort (12 ; 13) agit sur le récipient pour produit (2) dans la direction distale (V), de préférence par l'intermédiaire du piston (3).
